# EUROPEAN PATENT APPLICATION

(11) **EP 0 631 759 A1**
(43) Date of publication of application: **04.01.1995**
(21) Application number: 94250128.9
(22) Date of filing: 16.05.1994
(51) Int. Cl.: A61B 17/12, A61B 17/068

(54) **Clip-inducing apparatus for ligature clips, in particular for omega clips**

(30) Priority: 24.05.1993 DE 4317590
(71) Applicant: ETHICON INC., Somerville, New Jersey 08876-0151 (US)
(72) Inventor: Schulze, Dale, Lebanon Ohio 45036 (US); Höppner, Dirk, D-22399 Hamburg (DE)
(74) Representative: UEXKÜLL & STOLBERG

(57) **Abstract**

A Clip-inducing apparatus for omega clips (12), in particular for laparoscopic operations, consists of a tube-like rod system (1) at whose proximal end an operating device (2) and at whose distal end a clip-holding and clip-shaping mechanism are provided. The rod system (1) comprises an outer guide tube (16) in which there is arranged an inner sliding sleeve (17), displaceable coaxially to the outer guide tube (16), in which are located an elongated support rod (18) fixed relative to the outer guide tube (16) and an elongated slide (19) displaceable on the support rod (18). The proximal end of the outer guide tube (16) is firmly connected to the handle (3) of the operating device (2). The movable actuating lever (5) of the operating device (2) is in operational connection with the slide (19) and the sliding sleeve (17) at their proximal ends in such a way that, firstly, the sliding sleeve (17) and the slide (19) running ahead of the sliding sleeve (17) are displaced in the direction of the distal end, designed as an anvil (27), of the fixed support rod (18) and that, after the distal end of the slide (19), designed as a tip, has struck the anvil (27), a displacement of the sliding sleeve (17) in the same direction beyond the distal end of the support rod (18) and of the slide (19) is executed.

## Description

The invention relates to a clip-inducing apparatus for laparoscopic surgical operations, whereby the clips (or staples) used, made from a bendable filamentary material, are brought with the help of the clip-inducing apparatus in the application area into a closed shape firmly clamping the tissue located in between.

According to DE 40 24 106 a clip-inducing apparatus is described which consists of a tube-like rod system at the one, distal end of which a clip-holding and clip-shaping mechanism is located, while the other, proximal end displays an actuation mechanism.

The distal end of the clip-inducing apparatus is introduced into the patient's abdominal cavity through a cannula and positioned e.g. at the vessel to be tied. Through pressure on the actuation mechanism, the shaping of the ligature clip and thus the clamping of the vessel, e.g. an aorta, is effected via the tube-like rod system.

According to EP 0 150 826, an endoscopic apparatus for the induction of ligature clips is known. With this apparatus, the distal end is of forceps-like design for the reception and shaping of the clip. With the actuation mechanism located at the proximal end of the apparatus, the distal end is guided into the application area, the clip is laid around the vessel to be clamped and shaped by means of the forceps-like formation until the vessel is completely clamped.

The clips (or staples) used according to the known prior art correspond largely in their shape to the form of office staples. With modern minimally invasive operation techniques, surgical clips are used which are guided by means of a clip-inducing apparatus through a cannula to the operation area in the body. Since, with the clip-inducing apparatuses described, the clips carried along by the induction apparatus are advanced with their rectilinear base side crosswise to the longitudinal axis of the cannula and the cannula customarily used has a limited opening for the guiding through of instruments with a diameter of 5 or 10 mm, limits are also set to the size of the clips. The disadvantage of the described technique is thus that only clips of relatively small basic length and thus small circumference and small overall size are usable in endoscopic operations through cannulae. According to German Patent Application P 42 15 449.9, a new type of surgical clip, the so-called omega clip, was therefore created which is introducible with an induction apparatus through a cannula with pre-set diameter and shapable into a closed shape with a greater circumference than conventional clips. This omega clip is not usable with conventional clip-inducing apparatuses.

It is therefore the object of the invention to create a clip-inducing apparatus which has at its distal end a clip-holding and clip-shaping mechanism for omega clips which is triggerable via an actuation mechanism located at the proximal end.

A clip-inducing apparatus according to the preamble of claim 1, which is characterized by the following main features, serves to achieve this object:

The rod system consists of an outer guide tube in which there is arranged an inner sliding sleeve, displaceable coaxially to the outer guide tube, in which are located an elongated support rod fixed relative to the outer guide tube and an elongated slide displaceable on the support rod, whereby the clip-holding and clip-shaping mechanism comprised by sliding sleeve, support rod and slide project from the distal end of the outer guide tube in a manner guaranteeing the clip-holding and clip-shaping function of the clip-inducing apparatus.

The proximal end of the outer guide tube is firmly connected to the handle of the operating device and the movable actuating lever of the operating device is in active connection with the slide and the sliding sleeve at their proximal ends in such a way that, firstly, a joint displacement of the sliding sleeve and of the slide running ahead of the sliding sleeve in the direction of the distal end, designed as an anvil, of the fixed support rod and, after the distal end of the slide, designed as a tip, has struck the anvil, a displacement of the sliding sleeve in the same direction beyond the distal end of the support rod and of the slide is executable, the sliding sleeve and the slide being returnable to the starting position in proximal direction upon release of the actuating lever.

The invention will now be described in more detail with reference to an embodiment. The associated drawings show in
Fig. 1 a side view of the clip-inducing apparatus according to the invention,
Fig. 2 a section representation of the side view of the operating device,
Fig. 3 an enlarged representation of Fig. 2,
Fig. 4 the distal zone of the clip-inducing apparatus with fitted magazine for several omega clips,
Fig. 5 a plan view of Fig. 4,
Fig. 6 a - d views of chronologically successive shaping steps during the induction of a specific type of omega clips,

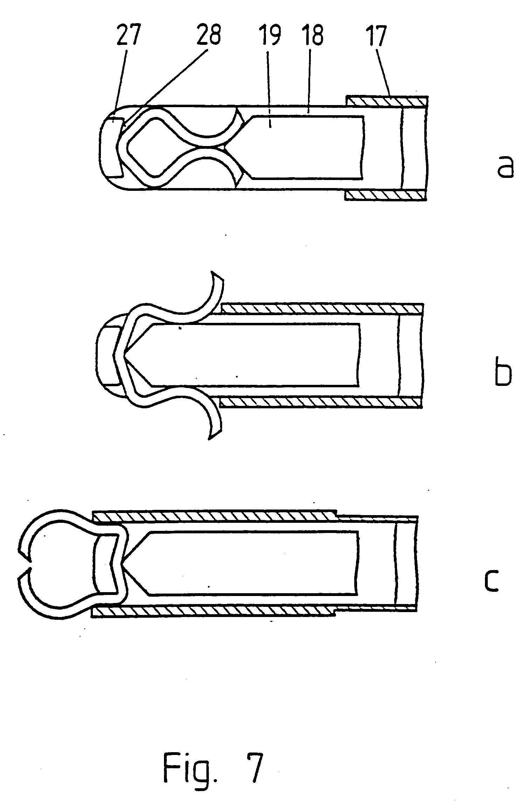

Fig. 7 a - c views of chronologically successive shaping steps during the induction of another type of omega clips.

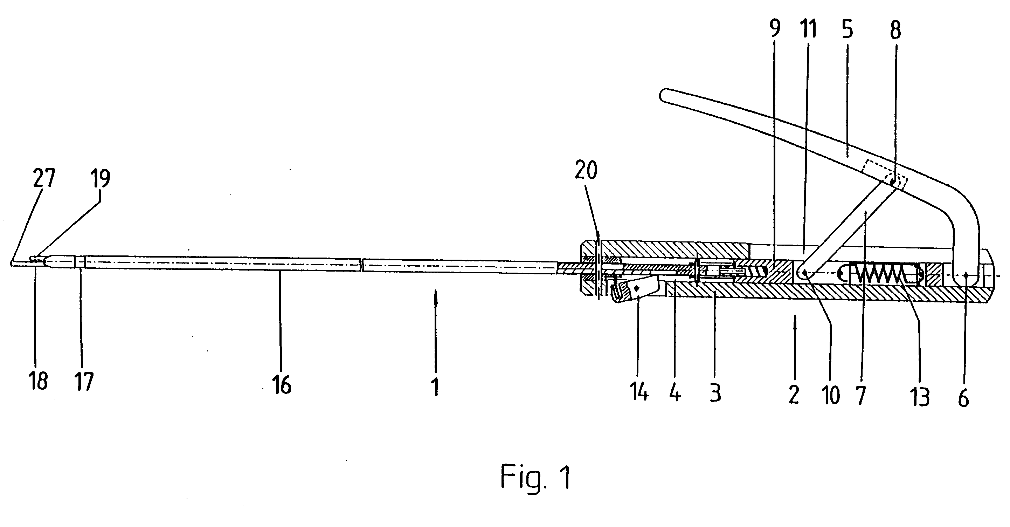

The clip-inducing apparatus represented in its entirety in Fig. 1 consists of an elongated rod system 1. This rod system 1 has several elements which guarantee, via an operating device 2 arranged at the proximal end, the holding and shaping of the omega clip at the distal end.

The operating device 2 consists of a sleeve-shaped handle 3 which has a guide bore 4 running in axial direction. At the end of the handle 3, an angled actuating lever 5 is housed swivellable on one side about a cylindrical pin 6. The free limb of the actuating lever 5 runs above the handle 3 in such a way that both the handle 3 and the actuating lever 5 can be grasped simultaneously by the operator's hand.

The transmission of the manual force onto the clip-inducing apparatus takes place via a knee-joint mechanism whose member 7 is housed pivotable on one side, in the central zone of the actuating lever 5, about a cylindrical pin 8 and on the other side, in a pressure rod 9 axially displaceable in the guide bore 4 of the handle 3, about a cylindrical pin 10, the member 7 being guided through a longitudinal slot 11 located in the handle 3. This longitudinal slot 11 located in the handle 3 is so designed that it guarantees the movement both of the member 7 and of the actuating lever 5 between defined end-positions, one end-position being determined by the readiness of the clip-inducing apparatus to receive an omega clip 12 and the other end-position by the completed shaping of the omega clip 12.

Accordingly, the clip-holding and clip-shaping elements are to be moved by the operating device 2.

The actuating lever 5 is held in its starting position by a tension spring 13 acting between the pressure rod 9 and the proximal end of the handle 3 if no manual force is acting on the actuating lever 5 and the stops 14 arranged at the handle 3 are not engaged with the clip-holding and clip-shaping elements, the return of the pressure rod 9 being limited by a spacer 15.

This position of the operating device 2 corresponds to the readiness position of the clip-inducing apparatus for receipt of an omega clip 12.

The clip-holding and clip-shaping elements are arranged in an outer guide tube 16 with a length necessary for laparoscopic surgical operations and a diameter permitting guidance through a cannula. This outer guide tube 16 is firmly connected with its one, proximal end to the handle 3 of the operating device 2, while the other, distal end of the guide tube 16 is open and permits the emergence of the clip-holding and clip-guiding elements.

Arranged coaxial to the outer guide tube 16 is an inner sliding sleeve 17 which accommodates an elongated support rod 18, fixed relative to the handle 3 and the outer guide tube 16, and an elongated slide 19, displaceable on the support rod 18. These clip-holding and clip-shaping elements emerge far enough from the distal end of the outer guide tube 16 to guarantee the clip-holding and clip-shaping function of the clip-inducing apparatus.

While the outer guide tube 16 and the support rod 18 are fixed to the handle 3 by a cylindrical pin 20, the sliding sleeve 17 and the slide 19 are in active connection with the pressure rod 9 of the operating device 2 so that the sliding sleeve 17 and the slide 19 experience a longitudinal displacement between the said end-positions.

There is a firm connection between the pressure rod 9 and the sliding sleeve 17, e.g. through welding, which guarantees a direct transmission of the longitudinal displacement of the pressure rod 9 onto the sliding sleeve 17. The slide 19 displaceable on the fixed support rod 18 is on the other hand in active connection with the pressure rod 9 indirectly via a compression spring 21. To this end, the pressure piece 9 is provided at its distal end with an axial blind bore 22 which accommodates both the proximal end of the sliding sleeve 17 and the proximal end of the slide 19, the compression spring 21 being arranged between the latter and the bore bottom.

The technologically necessary relative axial displaceability between the sliding sleeve 17 on the one hand and the slide 19 on the other is limited by a cylindrical pin 23, inserted in the slide 19, whose projecting ends are guided in opposing longitudinal holes 24 of a bush 25 which is firmly connected to the pressure rod 9 and surrounds the sliding sleeve 17. In the area of the oblong holes 24, the sliding sleeve 17 also has corresponding oblong holes 26.

To guarantee the technologically necessitated process of the induction of the omega clip 12, the support rod 18 carries at its distal end a protruding anvil 27 which has an angled bearing surface 28 for the tip of the omega clip 12 laid on the support rod 18. In the situation in which an omega clip 12 is laid in the clip-inducing apparatus, both the slide 19 and the sliding sleeve 17 are in an end-position which is given by the actuating lever 5 located in the starting position, the position of the sliding sleeve 17 vis-a-vis the position of the slide 19 being set back relative to the distal end of the clip-inducing apparatus. Through pressure on the actuating lever 5, the pressure rod 9 is moved in the direction of the distal end. The pressure rod 9 displaces the sliding sleeve 17 directly and the slide 19 indirectly via the compression spring 21 together in the same direction until the slide 19 presses the omega clip 12 against the bearing surface 28 of the anvil 27. As this pressing takes place against the elastic force of the compression spring 21, a secure holding of the unshaped omega clip 12 at the distal end of the clip-inducing apparatus is assured. The pressure on the actuating lever 5 is interrupted by the operator in this phase in which the described necessary holding force is applied.

The clip-inducing apparatus is guided in this state through the cannula into the application area in the inside of the body and positioned at the surgical object.

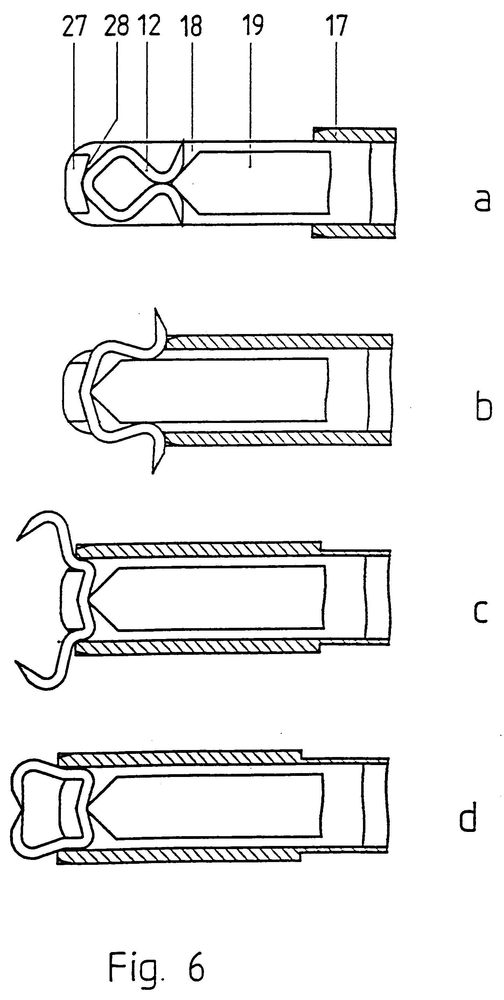

Through further pressure on the actuating lever 5 as per Fig. 6 a - d, the sliding sleeve 17 and the slide 19 are in turn moved by the pressure rod 9 in the direction of the distal end. The tipped end of the slide 19 slides between the limbs of the omega clip 12 and bends these apart until the feed movement of the slide 19 is ended by the fixed anvil 21. The result of further pressure on the actuating lever 5 is that the slide 17 switches with its proximal end against the elastic force of the compression spring 28 into the bore 22 of the pressure rod 9, while the sliding sleeve 17 is pushed beyond the distal end of the slide 17 and of the support rod 18 and the free limbs of the omega clip 12 are deformed forward into the closed shape.

For safely inducing the clip into the tissue, the situation of the clip-inducing apparatus according to Fig. 6 c (with the ends of the clip pointing forward) can be fixed by means of the stop 14 arranged in the handle 3 through form-locking engagement with the sliding sleeve 17.

Upon release of the actuating lever 5, the pressure rod 9 and thus the actuating lever 5 is guided back into the starting position by the tension spring 13. Through the direct connection between pressure rod 9 and sliding sleeve 17, the latter is also guided back directly into the starting position. During this rearward movement the compression spring 21 relaxes and the slide 19 is pulled into the starting position via the cylindrical pin 23 guided in the oblong holes 24 of the bush 25.

The induced omega clip 12 thus lies free and the clip-inducing apparatus can be removed from the body through the cannula in order to be reloaded if necessary.

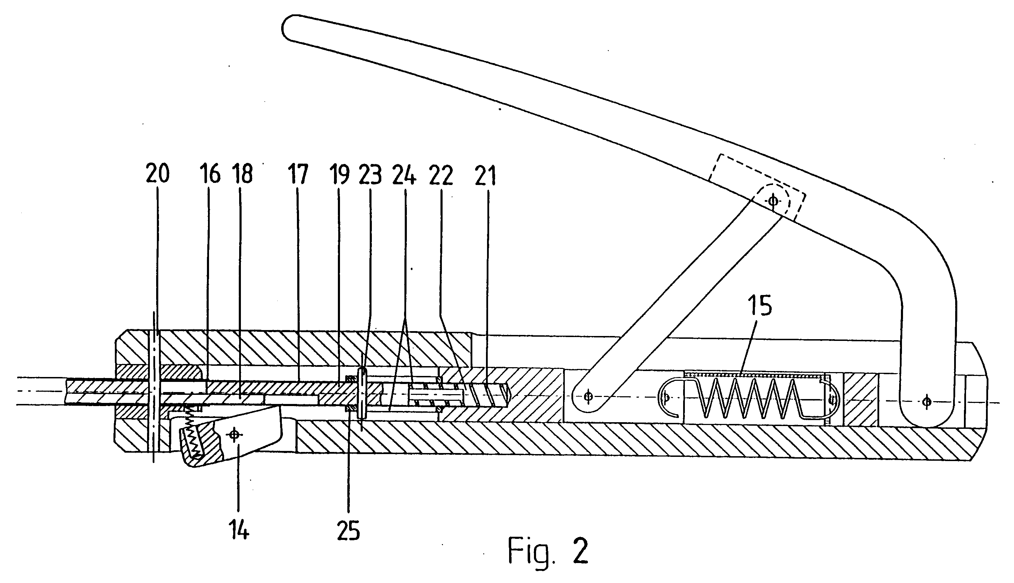

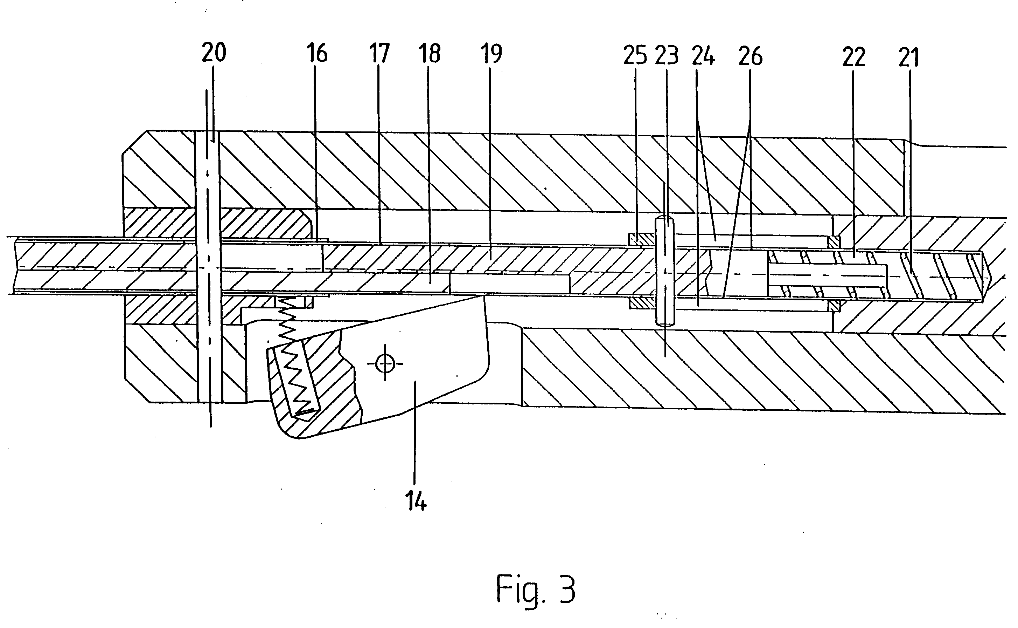

The embodiment described according to Fig. 1 - 3 assumes a single use of the apparatus according to the invention. This means that the clip-inducing apparatus must be reloaded outside the body after the induction of the omega clip 12. However, since the surgical procedure frequently requires several omega clips 12 to be induced, it is recommended that a stock of omega clips 12 be provided inside the clip-inducing apparatus, from which the clip-holding and clip-inducing mechanism can be charged in the vicinity of the surgical object.

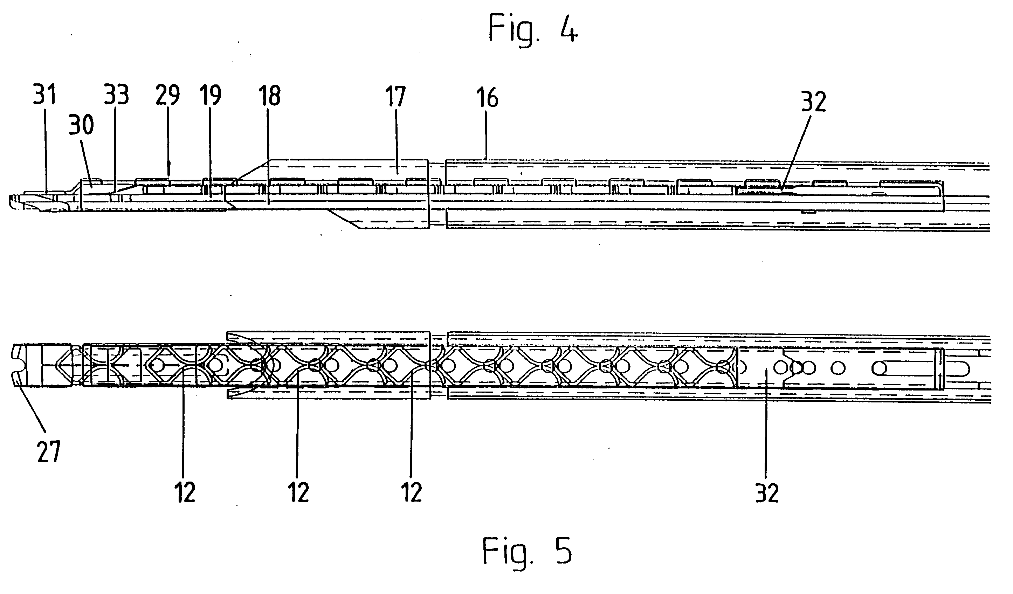

Figs. 4 and 5 show the equipping of the clip-inducing apparatus with a magazine 29 which is loaded with several omega clips 12 lying one behind the other.

The magazine 29 is formed of an elongated housing 30 which, inside the sliding sleeve 17, surrounds the support rod 18, the slide 19 and a series of omega clips 12 lying one behind the other on the slide 19. The omega clips 12 are so aligned that the tip of the clip engages between the limbs of the clip in front.

The housing 30 is provided at its distal outlet end with a holding-down device 31 which guarantees that the introduced omega clip 12 does not fall out of the clip-holding and clip-shaping mechanism.

At the opposite, proximal end the housing 30 has a spring-driven stop mechanism 32 with distal movement which distally shifts the clips being arranged in series by an amount corresponding to the distance between two clips and which thus sees to the feeding of the frontmost omega clip 12 into the space, left free by the slide 19 guided back into the starting position, between the anvil 27 and the tip of the slide 19. Through a spring 33, the omega clip 12 is directed into the described space after its forward movement and the withdrawal of the slide 19.

The induction of the omega clip 12 takes place as described for the example according to Fig. 1.

After the stock of preferably 10 omega clips 12 has been used up, the empty magazine 29 can be pulled out of the sliding sleeve 17 in the direction of the distal end of the clip-inducing apparatus, filled with new omega clips 12 and inserted in the clip-inducing apparatus.

## Claims

1. Clip-inducing apparatus for omega clips, in particular for laparoscopic operations, consisting of a tube-like rod system at whose one, proximal end an operating device and at whose other, distal end a clip-holding and clip-shaping mechanism are provided, **characterized in that** the rod system (1) comprises an outer guide tube (16) in which there is arranged an inner sliding sleeve (17), displaceable coaxially to the outer guide tube (16), in which are located an elongated support rod (18) fixed relative to the outer guide tube (16) and an elongated slide (19) displaceable on the support rod (18), whereby the clip-holding and clip-shaping mechanism comprised by the sliding sleeve (17), support rod (18) and slide (19) project from the distal end of the outer guide tube (16) in a manner guaranteeing the clip-holding and clip-shaping function of the clip-inducing apparatus, that the proximal end of the outer guide tube (16) is firmly connected to the handle (3) of the operating device (2) and the movable actuating lever (5) of the operating device (2) is in operational connection with the slide (19) and the sliding sleeve (17) at their proximal ends in such a way that, firstly, a displacement of the sliding sleeve (17) and of the slide (19) running ahead of the sliding sleeve (17) in the direction of the distal end, designed as an anvil (27), of the fixed support rod (18) and, after the distal end of the slide (19), designed as a tip, has struck the anvil (27), a displacement of the sliding sleeve (17) in the same direction beyond the distal end of the support rod (18) and of the slide (19) is executable, the sliding sleeve (17) and the slide (19) being guidable back into the starting position in proximal direction upon release of the actuating lever (5).

2. Clip-inducing apparatus according to claim 1, **characterized in that** a magazine (29) for stocking several omega clips (12) is arranged in the sliding sleeve (17) and consists of an elongated housing (30) which, inside the sliding sleeve (17), surrounds the support rod (18), the slide (19) and a series of omega clips (12) lying one behind the other on the slide (19), the omega clips (12) being so aligned that the tip of the clip engages between the limbs of the clip in front and the housing (30) is provided at its outlet end lying near to the distal end of the clip-inducing apparatus with a holding-down device (31) preventing an unintentional falling out of the introduced omega clip (12) from the clip-holding and clip-shaping mechanism, and at the opposite end of the housing (30) a spring-driven stop mechanism (32) with distal movement sees to the feeding of the front-most omega clip (12) into the space, left free by the slide (19) guided back into the starting position, between the anvil (27) and the tip of the slide (19), a spring (33) directing the frontmost omega clip (12) into the described space upon its forward movement.

3. Clip-inducing apparatus according to claim 1, **characterized in that** the operating device (2) consists of a sleeve-shaped handle (3) which has a guide bore (4) running in axial direction, an angled actuating lever (5) being housed swivellable on one side about a cylindrical pin (6) at the end of the handle (3) and the free limb of the actuating lever (5) running above the handle (3) in such a way that both the handle (3) and the actuating lever (5) can be grasped simultaneously by the operator's hand, and the transmission of the manual force onto the clip-inducing apparatus takes place via a member (7) which is housed pivotable on one side, in the central zone of the actuating lever (5), about a cylindrical pin (8) and on the other side, in a pressure rod (9) axially displaceable in the guide bore (4) of the handle (3), about a cylindrical pin (10), the member (7) being guided through a longitudinal slot (11) located in the handle (3) and the pressure rod (9) being in active connection with the sliding sleeve (17) and the slide (19).

4. Clip-inducing apparatus according to claim 3, **characterized in that** the swivellability both of the member (7) and of the actuating lever (5) between defined end-positions isguaranteed, one end-position being determined by the readiness of the clip-inducing apparatus to receive an omega clip (12) and the other end-position by the completed shaping of the omega clip (12).

5. Clip-inducing apparatus according to claim 3, **characterized in that** the sliding sleeve (17) is directly connected to the pressure rod (9) and the slide (19) is indirectly in active connection with the pressure rod (9) via a coaxially acting compression spring (21), means being provided between the sliding sleeve (17) on the one hand and the slide (19) on the other for limiting the relative displaceability among each other.

6. Process for inducing omega clips, in particular for laparoscopic operations, by means of a clip-inducing apparatus according to claim 1, characterized by the following steps:
- insertion of the omega clip (12) into the space formed between the anvil (27) and the tip of the slide (19) when the operating device (2) is in the starting position,
- advancing of the sliding sleeve (17) and of the slide (19) running ahead of the sliding sleeve (17) through the exertion of pressure on the actuating lever (5) until the fixing of the omega clip (12) between the bearing surface (28) of the anvil (27) and the tip of the slide (19), positioning of the clip-inducing apparatus with its clip-holding and clip-shaping mechanism located at the distal end at the surgical object,
- further advancing of the sliding sleeve (17) and of the slide (19) through continued exertion of pressure on the actuating lever (5), during which the tip of the slide (19) slides between the limbs of the omega clip (12) and bends these open until the advance of the slide (19) is stopped by the anvil (21),
- further advancing of the sliding sleeve (17) through continued exertion of pressure on the actuating lever (5), the distal end of the sliding sleeve (17) being pushed beyond the distal end of the support rod (18) and of the slide (17) and the limbs of the omega clip (12) being bent forward into a shape embracing the surgical object,
- release of the operating lever (5) and thus guiding back of the sliding sleeve (17) and of the slide (19) into the starting position permitting a loading of the clip-inducing apparatus with a next omega clip (12).
